Europäisches Patentamt

European Patent Office

Office européen des brevets

(11) Veröffentlichungsnummer: **0 302 192**
**A1**

## (12) EUROPÄISCHE PATENTANMELDUNG

(21) Anmeldenummer: **88108443.8**

(22) Anmeldetag: **27.05.88**

(51) Int. Cl.4: **A61N 1/16**

(30) Priorität: **16.07.87 DE 8709761 U**

(43) Veröffentlichungstag der Anmeldung:
**08.02.89 Patentblatt 89/06**

(84) Benannte Vertragsstaaten:
**AT BE CH DE ES FR GB GR IT LI LU NL SE**

(71) Anmelder: **Oehme, Rainer**
**Moorstrasse 10**
**D-3033 Schwarmstedt(DE)**

(72) Erfinder: **Oehme, Rainer**
**Moorstrasse 10**
**D-3033 Schwarmstedt(DE)**

(74) Vertreter: **Schlagwein, Udo, Dipl.-Ing.**
**Anwaltsbüro Ruppert & Schlagwein**
**Frankfurter Strasse 34**
**D-6350 Bad Nauheim(DE)**

(54) **Vorrichtung zur Behandlung von Lebewesen und Pflanzen durch Pyramidenenergie.**

(57) Bei einer Vorrichtung zur Behandlung von Lebewesen und Pflanzen durch Pyramidenenergie erfolgt der Energieabgriff an entlang der vier Kanten der Pyramide (6) galvanisch getrennten Energieaufnahmeleitungen (12, 13). Da die Energieabstrahlung an den Pyramidenkanten besonders stark ist, kann von solchen Energieaufnahmeleitungen (12, 13) besonders viel Energie aufgenommen werden. Solche Energieaufnahmeleitungen (12, 13) sind wesentlich billiger herstellbar.

Fig. 2

## Vorrichtung zur Behandlung von Lebewesen und Pflanzen durch Pyramidenenergie

Die Erfindung bezieht sich auf eine Vorrichtung zur Behandlung von Lebewesen und Pflanzen durch Pyramidenenergie, bei welcher außenseitig an der Pyramide zumindest ein Energieabgriff vorgesehen ist, von dem eine elektrische Leitung zu einer Chakra-Elektrode führt. Eine solche Vorrichtung ist Gegenstand der DE-OS 35 30 841.

Bei der bekannten Vorrichtung erfolgt der Energieabgriff an der Mantelfläche eines als Schwingtopf wirkenden Bechers, der von oben her auf die Pyramide aufgesetzt ist. Die Tatsache, daß mittels der bekannten Vorrichtung zwischen den Chakraelektroden Energien vorhanden sind, konnte durch Messung der Radioaktivität einer Wasserprobe nachgewiesen werden, indem eine gleiche Probe außerhalb der Vorrichtung angeordnet und die Radioaktivität über die Zeit gemessen wurde. Bei der Probe zwischen den Chakra-Elektroden zeigte sich im Gegensatz zur anderen Probe innerhalb kurzer Zeit ein rascher Abfall der Radioaktivität. Hiervon abgesehen wird die vorbekannte Vorrichtung zur Therapie des Menschen eingesetzt und hat zu erstaunlichen Heilerfolgen geführt.

Nachteilig bei der bekannten Vorrichtung ist es, daß diese durch den aufgesetzten Becher, der aus einer teuren Messinglegierung oder anderen, teilweise noch teureren Werkstoffen bestehen muß, relativ teuer in der Herstellung ist, so daß sie aus Kostengründen noch nicht die Verbreitung finden konnte, die aufgrund ihrer guten therapeutischen Wirkung angemessen wäre.

Der Erfindung liegt die Aufgabe zugrunde, eine Vorrichtung der eingangs genannten Art derart zu gestalten, daß die Pyramidenenergie mit möglichst geringem Aufwand an der Pyramide abgegriffen werden kann und die eine möglichst hohe Wirksamkeit aufweist.

Diese Aufgabe wird erfindungsgemäß dadurch gelöst, daß der Energieabgriff an einer entlang einer Kante der Pyramide verlaufenden und von der Pyramide galvanisch getrennten Energieaufnahmeleitung vorgesehen ist.

Da die Energieabstrahlung an den Pyramidenkanten besonders stark ist, kann von solchen Energieaufnahmeleitungen besonders viel Energie aufgenommen werden. Die Energieaufnahmeleitungen, welche durch einfache, im geringen Abstand von den Pyramidenkanten verlaufende, nicht isolierte Kupferdrähte gebildet sein können, sind wesentlich billiger herstellbar als der bekannte, als Schwingtopf wirkende, auf die Pyramide aufgesetzte Becher. Daher ist eine Pyramidenenergieanlage nach der Erfindung erheblich kostengünstiger herstellbar als die bekannte Vorrichtung.

Eine therapeutisch vorteilhaft wirkende Vorrichtung gemäß der Erfindung zeichnet sich dadurch aus, daß an den beiden Enden der Energieaufnahmeleitung ein Energieabgriff vorgesehen ist und der obere Energieabgriff mit einer oberen Chakra-Elektrode und der untere Energieabgriff mit einer unteren Chakra-Elektrode verbunden ist.

Ganz besonders vorteilhaft ist es, wenn entlang jeder Kante der Pyramide eine Energieaufnahmeleitung mit zwei Energieabgriffen vorgesehen ist und die oberen Energieabgriffe mit vier Einzelelektroden der oberen Chakra-Elektrode und die unteren Energieabgriffe mit vier Einzelelektroden der unteren Chakra-Elektrode verbunden sind. Durch diese Gestaltung kann mit noch besseren therapeutischen Wirkungen gerechnet werden, da an den einzelnen Kanten unterschiedliche Energien abgestrahlt werden, die ohne gegenseitige Beeinflussung zu den Einzelelektroden geführt werden. Hierzu im Gegensatz ist bei dem als Schwingtopf wirkenden Becher eine gegenseitige Beeinflussung der vom Becher aufgenommenen Energien zu erwarten.

Da die Energien rechtwinklig zur Pyramidenkante abgestrahlt werden, ist es vorteilhaft, wenn die Energieabgriffe in Höhe der Pyramidenspitze und der Pyramidenbasis jeweils am Ende einer rechtwinkligen Umbiegung der Energieaufnahmeleitung vorgesehen sind.

Die weitergeleitete Energie erhält einen den Chakrazentren entsprechenden Drall, wenn die Umbiegungen nach Art eines Korkenziehers rechtsdrehend gewendelt sind. Zur Förderung dieses Effektes kann man die Abgangsleitungen als rechtsgewendelte Leiter ausbilden oder die Abgangsleitungen als Ganzes rechtsdrehend gewunden zu den Chakra-Elektroden führen.

Die Engerieaufnahmeleitungen können auf sehr einfache Weise in einem festgelegten Abstand zur jeweiligen Pyramidenkante gehalten werden, wenn gemäß einer anderen Ausgestaltung der Erfindung die Energieaufnahmeleitungen jeweils mit einem Teilbereich ihrer beiden durch die Umbiegungen gebildeten Winkel in jeweils einem Schlitz eines Isolierstückes an der Pyramidenspitze und der Pyramidenbasis gehalten sind.

Hierbei ist es vorteilhaft, wenn die an der Basis der Pyramide an jeder Kante vorgesehenen Isolierstücke jeweils mit einer rechtwinkligen Ausnehmung über eine Ecke der Pyramide greifen und in ihrer Oberfläche den die Energieaufnahmeleitung aufnehmenden Schlitz haben.

Die von der Pyramidenspitze abgestrahlte Energie kann auf einfache Weise ebenfalls aufgefangen und dadurch genutzt werden, wenn in dem auf der Spitze der Pyramide vorgesehenen Isolierstück eine galvanisch von der Pyramide getrennte, koaxial zur Pyramidenachse ausgerichtete und nach Art eines Korkenziehers rechtsdrehend gewendelte Energieaufnahmeelektrode vorgesehen ist.

Besonders einfach ist das obere Isolierstück gestaltet, wenn es ein Zylinder mit einer koaxialen Bohrung zur Aufnahme der Energieaufnahmeelektrode und mit vier radialen Schlitzen für die Energieaufnahmeleitungen ist und wenn die der Pyramidenspitze zugewandte Stirnfläche des Isolierstückes entsprechend der Pyramidenneigung als Innenkegel ausgebildet ist.

Falls die Energie an der Spitze der Pyramide mit aufgenommen wird, dann ist die Wirkung der Pyramidenenergieanlage besonders groß, wenn gemäß einer anderen Ausgestaltung der Erfindung die obere Chakra-Elektrode zusätzlich zu den vier mit den oberen Energieabgriffen verbundenen Einzelelektroden eine mittlere Einzelelektrode hat, welche mit der Energieaufnahmeelektrode der Pyramidenspitze verbunden ist und entsprechend der Pyramidenhöhe tiefer nach unten reicht als die übrigen Einzelelektroden. Abgesehen von dem einfacheren Energieabgriff ist die erfindungsgemäße Vorrichtung im Vergleich zu den bisher bekannten Vorrichtungen auch deshalb kostengünstiger herstellbar, weil die obere Chakra-Elektrode nur noch fünf und die untere Chakra-Elektrode nur noch vier Einzelelektroden benötigt.

Für die therapeutische Behandlung des Menschen ist es vorteilhaft, wenn alle Einzelelektroden der beiden Chakraelektroden mit denjenigen Energieabgriffen verbunden sind, welche mit den Einzelelektroden fluchten, wenn man sich die Pyramide mit ihrer Spitze nach unten wei send zwischen den Chakraelektroden denkt.

Die Erfindung läßt zahlreiche Ausführungsformen zu. Zur weiteren Verdeutlichung ihres Grundprinzips ist eine davon in der Zeichnung dargestellt und wird nachfolgend beschrieben. Die Zeichnung zeigt in

Fig. 1 eine Seitenansicht einer kompletten, neuerungsgemäßen Vorrichtung,

Fig. 2 eine Seitenansicht der Vorrichtung im Bereich ihrer Pyramide,

Fig. 3 eine Draufsicht auf die Anordnung nach der Figur 2, jedoch ohne Energieaufnahmeleitungen,

Fig. 4 eine Seitenansicht der oberen Chakra-Elektrode,

Fig. 5 einen horizontalen Schnitt durch die obere Chakra-Elektrode,

Fig. 6 eine Seitenansicht der unteren Chakraelektrode,

Fig. 7 einen horizontalen Schnitt durch die untere Chakra-Elektrode,

Fig. 8 eine schematische Darstellung der Verdrahtung der Vorrichtung,

Fig. 9 einen Längsschnitt durch ein oberes Isolierstück der Vorrichtung,

Fig. 10 eine Draufsicht auf das obere Isolierstück,

Fig. 11 eine Draufsicht auf ein unteres Isolierstück,

Fig. 12 einen Vertikalschnitt durch das untere Isolierstück.

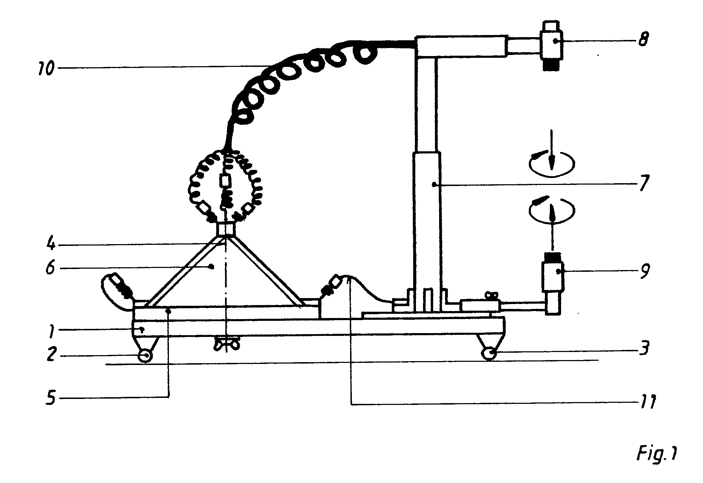

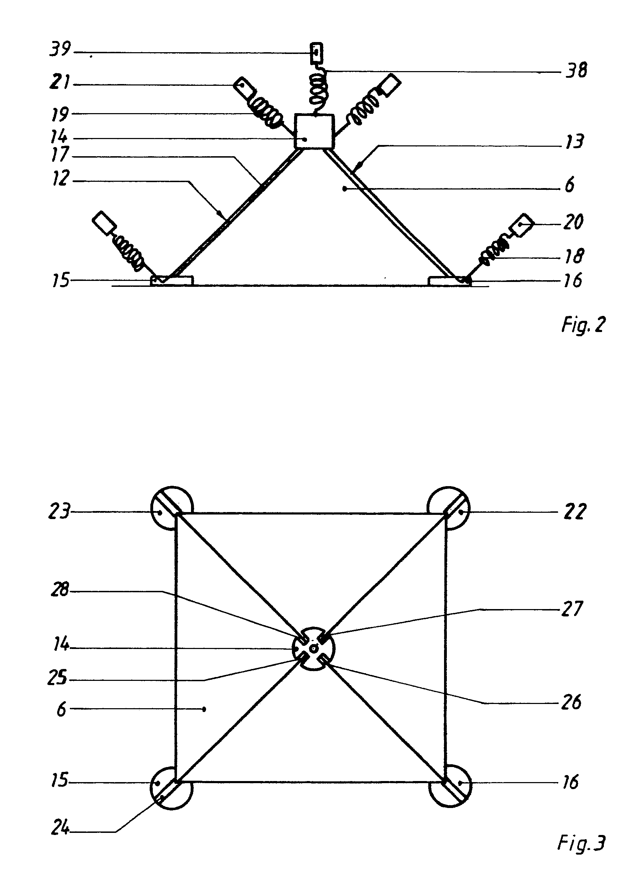

Die in Figur 1 dargestellte Vorrichtung hat eine Grundplatte 1 mit Laufrädern 2, 3. Auf dieser Grundplatte 1 ist um eine senkrechte Achse 4 verdrehbar eine Auflage 5 angeordnet, auf der eine Pyramide 6 aus beispielsweise Kupfer steht. Vor der Auflage 5 ist ein Stativ 7 auf der Grundplatte 1 befestigt, welches eine obere Chakra-Elektrode 8 und eine untere Chakra-Elektrode 9 haltert. Die Pyramidenenergie wird den beiden Chakra-Elektroden 8, 9 über Leitungen 10, 11 zugeführt. Wichtig für die Erfindung ist die Art der Energieaufnahme von der Pyramide 6. Diese ergibt sich am deutlichsten aus den Figuren 2 und 3.

Die Figur 2 zeigt, daß parallel zu den Kanten der Pyramide 6 jeweils eine Energieaufnahmeleitung 12, 13 verläuft, welche an der Pyramidenspitze von einem oberen, allen vier Energieaufnahmeleitungen 12, 13 gemeinsamen Isolierstück 14 und an den Ecken der Basis der Pyramide 6 jeweils durch ein unteres Isolierstück 15, 16 gehalten sind. Jede Energieaufnahmeleitung 12, 13 besteht aus einem parallel der Pyramidenkante verlaufenden, nicht isolierten Drahtabschnitt 17 und einer rechtwinkligen Umbiegung 18, 19, die korkenzieherartig, rechtsdrehend gewendelt ist und an ihrem Ende jeweils einen Steckanschluß 20, 21 hat.

Das obere Isolierstück 14 der Pyramide 6 trägt koaxial zur Pyramide 6 eine Energieaufnahmeelektrode 38, welches aus einem rechtsdrehend gewendelten, korkenzieherartigen und in Haupterstreckungsrichtung nach oben weisenden Drahtstück gebildet ist, an dessen Ende als Energieabgriff ebenfalls ein Steckanschluß 39 vorgesehen ist.

Die Figur 3 zeigt, daß zusätzlich zu den Isolierstücken 15, 16 auch an den beiden anderen Ecken der Pyramide 6 entsprechende Isolierstücke 22, 23 vorgesehen sind. Diese Isolierstücke 15, 16, 22, 23 haben in ihrer Oberseite jeweils einen Schlitz, beispielsweise einen Schlitz 24 im Isolierstück 15, in den jeweils eine Energieaufnahmeleitung, zum Beispiel die Energieaufnahmeleitung 17, mit einem Teilbereich des durch die Umbiegung 18 gebildeten Winkels sitzt. Entsprechende Schlitze 25 - 28 sind in radialer Ausrichtung in der Mantelfläche des oberen Isolierstückes 14 vorgesehen, in die der jeweils obere Winkel jeder Energieaufnahmeleitung 12, 13 sitzt.

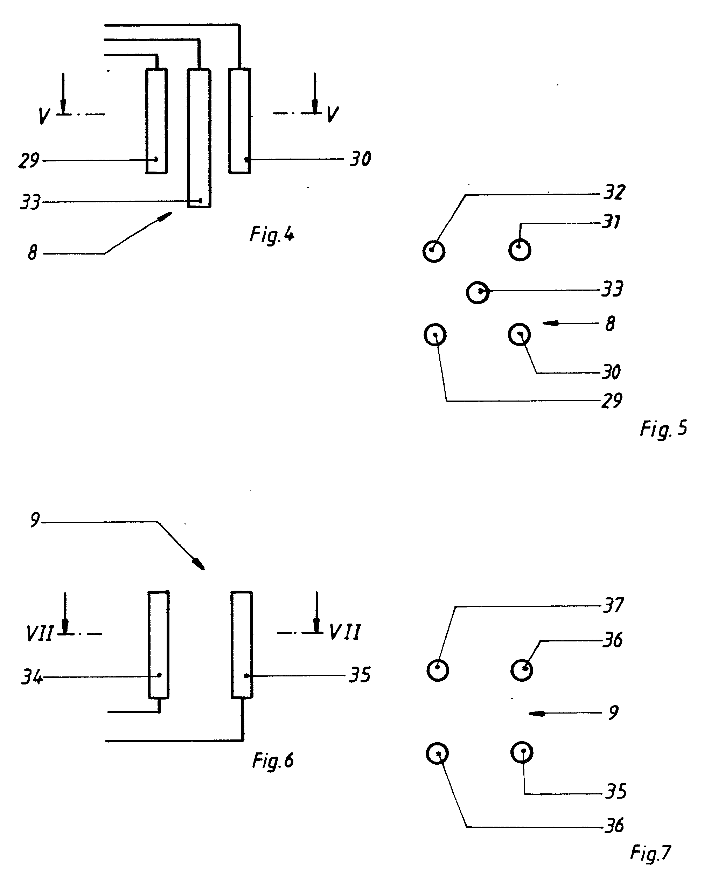

Die Figuren 4 und 5 lassen erkennen, daß die obere Chakra-Elektrode 8 aus insgesamt fünf Einzelelektroden 29 - 33 besteht, wobei die mittlere Einzelelektrode 33 um die Höhe der Pyramide 6 weiter nach unten reicht als die übrigen Einzelelektroden 29, 30, 31, 32.

Die in den Figuren 6 und 7 gezeigte untere Chakra-Elektrode 9 besteht aus vier Einzelelektroden 34 - 37, welche mit den Einzelelektroden 29 - 32 fluchten.

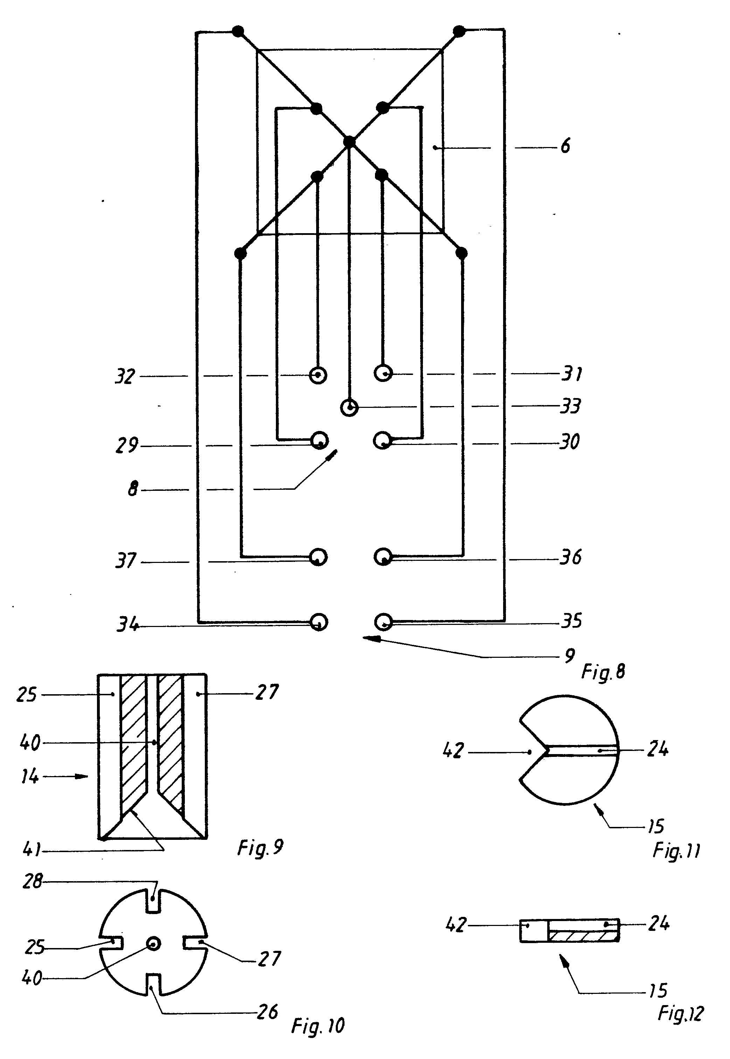

Die Figur 8 verdeutlicht, wie die Einzelelektroden 29 -37 mit den Energieabgriffen der Pyramide 6 verbunden sind. Die jeweils oberen Energieabgriffe der entlang der Pyramidenkanten geführten Energieaufnahmeleitungen 12, 13 und der Energieabgriff der Energieaufnahmeelektrode 38 auf der Spitze der Pyramide 6 sind mit der oberen Chakra-Elektrode 8 und die übrigen Energieabgriffe, also die vier an der Unterseite der Energieaufnahmeleitungen 12, 13 vorgesehenen Energieabgriffe, sind mit der unteren Chakra-Elektrode 9 verbunden. Denkt man sich die Pyramide 6 mit der Spitze nach unten weisend zwischen den Chakra-Elektroden 8, 9, dann führen alle Verbindungsleitungen ohne sich zu überkreuzen zu den jeweiligen Einzelelektroden 29 - 37.

Die Figuren 9 und 10 verdeutlichen die Gestaltung des oberen Isolierstückes 14. Man erkennt, daß koaxial durch es hindurch eine Bohrung 40 führt, die an der Unterseite in einen Innenkegel 41 mündet, der so bemessen ist, daß sich das Isolierstück 14 gut auf die Spitze der Pyramide 6 setzen läßt. Die Figur 9 zeigt weiterhin die zwei Schlitze 25, 27 und Figur 10 alle vier radialen Schlitze 25 - 28.

Die Figuren 11 und 12 zeigen am Beispiel des unteren Isolierstückes 15, daß die unteren Isolierstücke 15, 16, 22, 23 jeweils eine winkelförmige Ausnehmung 42 aufweisen, mit der sie um eine Ecke der Pyramide 6 zu greifen vermögen. Weiterhin zeigen die Figuren 11 und 12 den Schlitz 24, in den die Energieaufnahmeleitung 12 mit einer Ecke eingesetzt wird.

### Auflistung der verwendeten Bezugszeichen

1 Grundplatte
2 Laufrad
3 Laufrad
4 Achse
5 Auflage
6 Pyramide
7 Stativ
8 Chakra-Elektrode
9 Chakra-Elektrode
10 Leitung
11 Leitung
12 Energieaufnahmeleitung
13 Energieaufnahmeleitung
14 Isolierstück
15 unteres Isolierstück
16 Isolierstück
17 Drahtabschnitt
18 Umbiegung
19 Umbiegung
20 Steckanschluß
21 Steckanschluß
22 Isolierstück
23 Isolierstück
24 Schlitz
25 Schlitz
26 Schlitz
27 Schlitz
28 Schlitz
29 Einzelelektrode
30 Einzelelektrode
31 Einzelelektrode
32 Einzelelektrode
33 Einzelelektrode
34 Einzelelektrode
35 Einzelelektrode
36 Einzelelektrode
37 Einzelelektrode
38 Energieaufnahmeelektrode
39 Steckanschluß
40 Bohrung
41 Innenkegel
42 Ausnehmung

### Ansprüche

1. Vorrichtung zur Behandlung von Lebewesen und Pflanzen durch Pyramidenenergie, bei welcher außenseitig an der Pyramide zumindest ein Energieabgriff vorgesehen ist, von dem eine elektrische Leitung zu einer Chakra-Elektrode führt, dadurch gekennzeichnet, daß der Energieabgriff an einer entlang einer Kante der Pyramide (6) verlaufenden und von der Pyramide (6) galvanisch getrennten Energieaufnahmeleitung (12, 13) vorgesehen ist.

2. Vorrichtung nach Anspruch 1, dadurch gekennzeichnet, daß an den beiden Enden der Energieaufnahmeleitung (12, 13) ein Energieabgriff vorgesehen ist und der obere Energieabgriff zu einer oberen Chakra-Elektrode (8) und der untere Energieabgriff mit einer unteren Chakra-Elektrode (9) verbunden ist.

3. Vorrichtung nach Anspruch 1 oder 2, dadurch gekennzeichnet, daß entlang jeder Kante der Pyramide (6) eine Energieaufnahmeleitung (12, 13) mit zwei Energieabgriffen vorgesehen ist und die oberen Energieabgriffe mit vier Einzelelektroden (29 - 32) der oberen Chakra-Elektrode (8) und die unteren Energieabgriffe mit vier Einzelelektroden (34 - 37) der unteren Chakra-Elektrode (9) verbunden sind.

4. Vorrichtung nach Anspruch 2, dadurch gekennzeichnet, daß die Energieabgriffe in Höhe der Pyramidenspitze und der Pyramidenbasis jeweils am Ende einer rechtwinkligen Umbiegung (18, 19) der Energieaufnahmeleitung (12, 13) vorgesehen sind.

5. Vorrichtung nach Anspruch 3, dadurch gekennzeichnet, daß die Umbiegungen (18, 19) nach Art eines Korkenziehers rechtsdrehend gewendelt sind.

6. Vorrichtung nach einem oder mehreren der vorangehenden Ansprüche, dadurch gekennzeichnet, daß die Energieaufnahmeleitungen (12, 13) jeweils mit einem Teilbereich ihrer beiden durch die Umbiegungen (18, 19) gebildeten Winkel in jeweils einen Schlitz (25 - 28; 24) eines Isolierstückes (14) an der Pyramidenspitze und der Pyramidenbasis gehalten sind.

7. Vorrichtung nach Anspruch 5, dadurch gekennzeichnet, daß die Isolierstücke (15, 16, 22, 23) an der Basis der Pyramide (6) jeweils mit einer rechtwinkligen Ausnehmung (42) über eine Ecke der Pyramide (6) greifen und in ihrer Oberseite jeweils den die Energieaufnahmeleitung (12, 13) aufnehmenden Schlitz (24) haben.

8. Vorrichtung nach einem oder mehreren der vorangehenden Ansprüche, dadurch gekennzeichnet, daß in dem auf der Spitze der Pyramide (6) angeordneten Isolierstück (14) eine galvanisch von der Pyramide (6) getrennte, koaxial zur Pyramidenachse ausgerichtete unt nach Art eines Korkenziehers rechtsdrehend gewendelte Energieaufnahmeelektrode (38) vorgesehen ist.

9. Vorrichtung nach einem oder mehreren der vorangehenden Ansprüche, dadurch gekennzeichnet, daß das obere Isolierstück (14) ein Zylinder mit einer koaxialen Bohrung (40) zur Aufnahme der Energieaufnahmeelektrode (38) und mit vier radialen Schlitzen (25 - 28) für die Energieaufnahmeleitungen (12, 13) ist und daß die der Pyramidenspitze zugewandte Stirnfläche des Isolierstückes (14) entsprechend der Pyramidenneigung als Innenkegel (41) ausgebildet ist.

10. Vorrichtung nach einem oder mehreren der vorangehenden Ansprüche, dadurch gekennzeichnet, daß die obere Chakra-Elektrode (8) zusätzlich zu den vier mit den oberen Energieabgriffen verbundenen Einzelelektroden (29 -32) eine mittlere Einzelelektrode (33) hat, welche mit der Energieaufnahmeelektrode (38) der Pyramidenspitze verbunden ist und entsprechend der Pyramidenhöhe tiefer nach unten reicht als die übrigen Einzelelektroden (29 -32).

11. Vorrichtung nach einem oder mehreren der vorangehenden Ansprüche, dadurch gekennzeichnet, daß alle Einzelelektroden 29 - 35) der beiden Chakraelektroden (8 - 9) mit denjenigen Energieabgriffen verbunden sind, welche mit den Einzelelektroden 29 - 37) fluchten, wenn man sich die Pyramide (6) mit ihrer Spitze nach unten weisend zwischen den Chakraelektroden (8, 9) denkt.

*Fig. 1*

Fig. 2

Fig. 3

Fig.4

Fig. 5

Fig.6

Fig.7

EP 0 302 192 A1

32
31
33
29
30
8
37
36
34
35
9

Fig. 8

25      27
40
14
41

Fig. 9

28
25
40
27
26

Fig. 10

42      24
15

Fig. 11

42      24
15

Fig. 12

| | **EINSCHLÄGIGE DOKUMENTE** | | EP 88108443.8 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | DE - A1 - 3 433 292 (REHM) | 1 | A 61 N 1/16 |
| A | * Zusammenfassung; Patentansprüche 1,2; Fig. c * | 3 | |
| | -- | | |
| A | DE - A1 - 3 006 191 (OHRESSER) | 1 | |
| | * Anspruch 1; Fig. * | | |
| | -- | | |
| A | FR - A1 - 2 264 406 (FANTUZZI) | 1 | |
| | * Seite 3, Zeilen 1-3, 7-13; Fig. 1 * | | |
| | -- | | |
| A | DE - A1 - 3 416 157 (ROTH) | 1,3,5 | |
| | * Fig. 1,2 * | | |
| | -- | | RECHERCHIERTE SACHGEBIETE (Int. Cl.4) |
| A | FR - A - 1 057 531 (SANCHEZ) | 1,3, 5-8 | |
| | * Fig. 2 * | | A 61 N |
| | -- | | |
| A | FR - A - 1 108 193 (CARL) | 1,3,5 | |
| | * Fig. 1,3 * | | |
| | ---- | | |

Der vorliegende Recherchenbericht wurde für alle Patentansprüche erstellt.

| Recherchenort | Abschlußdatum der Recherche | Prüfer |
|---|---|---|
| WIEN | 22-11-1988 | NEGWER |